# EUROPÄISCHE PATENTSCHRIFT

(11) **EP 1 513 838 B1**
(45) Veröffentlichungstag und Bekanntmachung des Hinweises auf die Patenterteilung: **26.07.2006**
(21) Anmeldenummer: 03720568.9
(22) Anmeldetag: 16.05.2003
(51) Int. Cl.: C07D 451/06

(54) **TECHNISCHES VERFAHREN ZUR HERSTELLUNG VON TROPENOL**
TECHNICAL METHOD FOR PRODUCING TROPENOL
PROCEDE TECHNIQUE DE FABRICATION DE TROPENOL

(30) Priorität: 31.05.2002 DE 10224091
(43) Veröffentlichungstag der Anmeldung: 16.03.2005
(73) Patentinhaber: Boehringer Ingelheim Pharma GmbH & Co.KG, 55216 Ingelheim am Rhein (DE)
(72) Erfinder: BANHOLZER, Rolf, 70597 Stuttgart (DE); BODENBACH, Gisela, 88444 Ummendorf (DE); MATHES, Andreas, 55437 Ockenheim (DE); MEISSNER, Helmut, 55218 Ingelheim (DE); SPECHT, Peter, 55437 Ober-Hilbersheim (DE)
(86) Internationale Anmeldenummer: PCT/EP2003/005158
(87) Internationale Veröffentlichungsnummer: WO 2003/101986

(56) Entgegenhaltungen:
- DATABASE CA [Online] CHEMICAL ABSTRACTS SERVICE, COLUMBUS, OHIO, US; CHU, GUOHUA ET AL: "Synthesis of 6,7-dehydrotropine" retrieved from STN Database accession no. 125:248172 XP002250968 & ZHONGGUO YAOWU HUAXUE ZAZHI (1996), 6(1), 7-10 ,

## Beschreibung

Die Erfindung betrifft ein neues, technisch anwendbares Herstellverfahren zur Darstellung von Tropenol, gegebenenfalls in Form seiner Säureadditionssalze.

### Hintergrund der Erfindung

Die Verbindung Tropenol ist aus dem Stand der Technik bekannt und weist die folgende chemische Struktur auf:

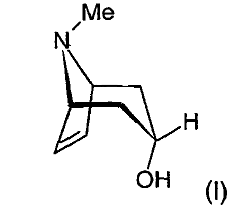

Die Verbindung kann als Ausgangsverbindung zur Darstellung pharmakologisch wertvoller Verbindungen Verwendung finden. Beispielsweise seien in diesem Zusammenhang genannt die Verbindungen Tiotropiumbromid, lpratropiumbromid oder auch BEA2108. Diese pharmakologisch wertvollen Substanzen sind durch die folgenden chemischen Strukturen gekennzeichnet:

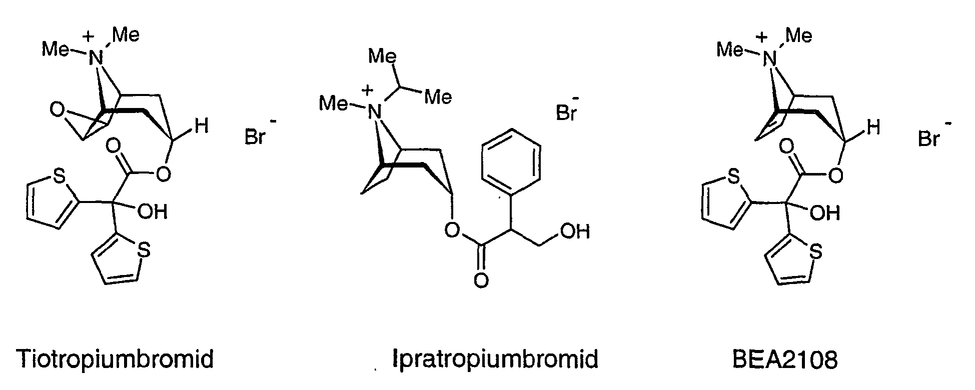

Aufgrund der hohen Wirksamkeit vorstehend genannter Verbindungen, ist es notwendig, selbige in möglichst hoher Reinheit mittels effizienter Syntheseverfahren zugänglich zu machen. Insbesondere das hohe Reinheitserfordernis, welches generell von therapeutisch zum Einsatz gelangenden Verbindungen erfüllt werden muss, setzt ein möglichst geringes Maß an Verunreinigungen in den Ausgangsverbindungen voraus. Werden bereits als Ausgangsverbindungen Materialien verwendet, die einen relativ hohen Anteil an Verunreinigung enthalten, so gestaltet sich die Aufreinigung des Endprodukts häufig schwierig, da eingangs eingebrachte Verunreinigungen auch in späteren Syntheseschritten häufig nicht mehr ohne weiteres, und wenn, dann lediglich unter großen Ausbeuteverlusten abtrennbar sind. Dies ist insbesondere dann der Fall, wenn sich die auftretenden Nebenprodukte bzw. Verunreinigungen von den jeweiligen Hauptprodukten strukturell nur in geringem Maße unterscheiden.

Vor diesem Hintergrund ist es Aufgabe der vorliegenden Erfindung, ein Syntheseverfahren bereitzustellen, welches den technischen Zugang zu Tropenol, vorzugsweise in Form eines seiner Säureadditionssalze, in guter Ausbeute und vor allem hoher Reinheit erlaubt.

### Detaillierte Beschreibung der Erfindung

Die vorstehend definierte Aufgabe wird durch die nachfolgend beschriebene Erfindung gelöst.

Die vorliegende Erfindung betrifft dementsprechend ein technisches Verfahren zur Herstellung von Tropenol der Formel (I) 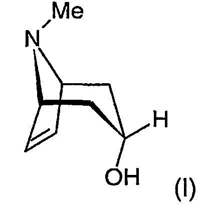 gegebenenfalls in Form seiner Säureadditionssalze, dadurch gekennzeichnet, dass eine Verbindung der Formel (II) 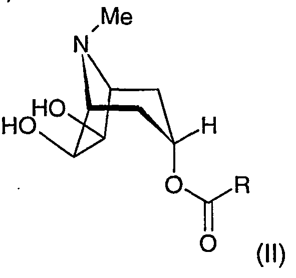 worin
- R: ein Rest ausgewählt aus C₁-C₄-Alkyl, C₂-C₆-Alkenyl und C₁-C₄-alkylen-Phenyl, die jeweils substituiert sein können durch Hydroxy oder C₁-C₄-Alkoxy, bedeutet,
gegebenenfalls in Form ihrer Säureadditionssalze sowie gegebenenfalls in Form ihrer Hydrate in einem geeigneten Lösemittel mit einem Formamid-acetal der Formel (III) 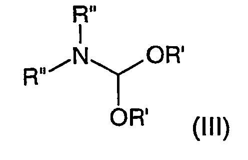 worin
R' C₁-C₄-Alkyl und R" ein Rest ausgewählt aus C₁-C₄-Alkyl und C₁-C₄-alkylen-Phenyl bedeuten, zu einer Verbindung der Formel IV 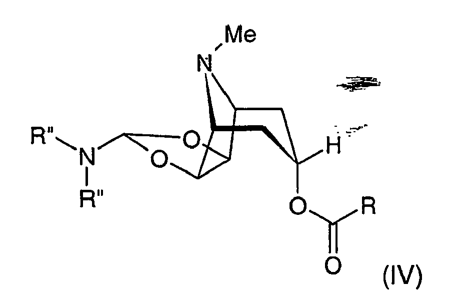 worin die Reste R, R' und R" die oben genannten Bedeutungen haben können, umsetzt, diese anschließend unter Decarboxylierung in einen Ester der Formel (V) 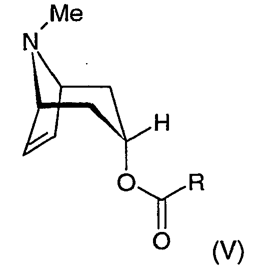 worin R die vorstehend genannten Bedeutungen haben kann, überführt und diesen abschließend zur Verbindung der Formel (I) verseift und diese durch Umsetzung mit einer geeigneten Säure gegebenenfalls in ein Säureadditionssalz überführt.

Bevorzugt betrifft die vorliegende Erfindung ein technisches Verfahren zur Herstellung von Tropenol der Formel (1), gegebenenfalls in Form seiner Säureadditionssalze, welches dadurch gekennzeichnet ist, dass eine Verbindung der Formel (II) worin R C₁-C₄-Alkyl oder C₂-C₄-Alkenyl bedeutet, gegebenenfalls in Form ihrer Säureadditionssalze sowie gegebenenfalls in Form ihrer Hydrate als Ausgangsmaterial verwendet wird und worin in dem eingesetzten Formamid-acetal der Formel (III) die Reste R' Methyl oder Ethyl und die Reste R" Methyl, Ethyl oder Propyl bedeuten.

Besonders bevorzugt betrifft die vorliegende Erfindung ein technisches Verfahren zur Herstellung von Tropenol der Formel (1), gegebenenfalls in Form seiner Säureadditionssalze, vorzugsweise in Form seines Hydrochlorids, welches dadurch gekennzeichnet ist, dass eine Verbindung der Formel (II) worin R 1-Propenyl, 2-Propenyl, 1-Buten-1-yl, 1-Buten-2-yl, 1-Buten-3-yl, 1-Buten-4-yl, 2-Buten-1-yl oder 2-Buten-2-yl bedeutet, gegebenenfalls in Form ihrer Säureadditionssalze sowie gegebenenfalls in Form ihrer Hydrate als Ausgangsmaterial verwendet wird und worin in dem eingesetzten Formamid-acetal der Formel (III) die Reste R' und R" Methyl oder Ethyl, bevorzugt Methyl bedeuten.

Besonders bevorzugt wird als Verbindung der Formel (II) jene Verbindung eingesetzt, in der R 2-Buten-2-yl bedeutet. Diese Verbindung ist auch unter dem Namen Meteloidin im Stand der Technik bekannt.

Unter C₁-C₄-Alkyl werden im Rahmen der vorliegenden Erfindung verzweigte oder unverzweigte Alkylgruppen mit bis zu 4 Kohlenstoffatomen verstanden. Beispielsweise seien genannt Methyl, Ethyl, n-Propyl, iso-Propyl, n-Butyl, sec-Butyl, iso-Butyl und tert-Butyl. Unter C₁-C₄-alkylen-Phenyl wird im Rahmen der vorliegenden Erfindung Phenyl verstanden, welches über eine verzweigte oder unverzweigte Alkylenbrücke mit bis zu 4 Kohlenstoffatomen verknüpft ist. Beispielsweise seien genannt Benzyl, Phenyl-2-ethyl-, Phenyl-1-ethyl-, Phenyl-3-propyl-, Phenyl-2-propyl- etc. Sowohl die C₁-C₄-Alkylgruppen als auch die C₁-C₄₋alkylen-Phenyl-gruppen können, sofern nicht anders definiert durch einen oder mehrere Hydroxy und/oder C₁-C₄-Alkyloxy-gruppen substituiert sein.

Unter C₂-C₆-Alkenyl werden im Rahmen der vorliegenden Erfindung verzweigte oder unverzweigte Alkenylgruppen mit 2 bis 6 Kohlenstoffatomen verstanden, die wenigstens eine Doppelbindung aufweisen. Beispielsweise seien genannt Vinyl, 1-Propenyl, 2-Propenyl, 1-Buten-1-yl, 1-Buten-2-yl, 1-Buten-3-yl, 1-Buten-4-yl, 2-Buten-1-yl, 2-Buten-2-yl, Butadien-1-yl, Butadien-2-yl etc.

Soweit nicht anders angegeben, werden im Rahmen der vorliegenden Erfindung unter Säureadditionssalzen die Salze verstanden, die mit den Säuren Salzsäure, Bromwasserstoff, Phosphorsäure, Schwefelsäure, Tetrafluoroborsäure oder Hexafluorophosphorsäure, bevorzugt Salzsäure oder Bromwasserstoffsäure gebildet werden.

Erfindungsgemäß kann zur Durchführung des erfindungsgemäßen Verfahrens zur Herstellung von Tropenol wie folgt vorgegangen werden.
In einem geeigneten Reaktionsgefäß wird die Verbindung der Formel (III) vorgelegt.

Pro Mol umzusetzende Verbindung der Formel (II) gelangt üblicherweise wenigstens 1 Mol der Verbindung (III) zum Einsatz. Vorzugsweise werden pro Mol der umzusetzenden Verbindung (III) zwischen 1,01 und 5,0 Mol, bevorzugt zwischen 1,1 und 4,0 Mol, besonders bevorzugt zwischen 1,5 und 3,0 Mol der Verbindung (III) vorgelegt. Unter Rühren erfolgt sodann die portionsweise Zugabe der Verbindung der Formel (II). Nach beendeter Zugabe wird die erhaltene Mischung vorzugsweise auf eine Temperatur von über 40°C, bevorzugt mehr als 50°C, besonders bevorzugt mehr als 60°C erwärmt. Im Laufe der Reaktion wird der Alkohol R'-OH frei. Dieser wird vorzugsweise destillativ dem Reaktionsgleichgewicht entzogen. Diese Destillation kann gegebenenfalls unter vermindertem Druck erfolgen. Wird als Verbindung der Formel (III) diejenige Verbindung eingesetzt, bei der R' Methyl bedeutet, wird bevorzugt eine Temperatur im Bereich von etwa 55-90°C, besonders bevorzugt von etwa 60-85°C eingestellt. Nach beendeter Umsetzung wird die gegebenenfalls im Überschuss eingesetzte Verbindung der Formel (III) destillativ, unter vermindertem Druck, entfernt. Hierzu wird die erhaltene Mischung vorzugsweise auf eine Temperatur von über 40°C, bevorzugt mehr als 50°C, erwärmt und ein Vakuum von 100 mbar oder weniger, vorzugsweise 60 mbar oder weniger, besonders bevorzugt 40 mbar oder weniger angelegt.

Der verbleibende Rückstand (Rohprodukt der allgemeinen Formel (IV)) wird anschließend unter Rühren in einem geeigneten Lösemittel, bevorzugt in einem polaren organischen Lösmittel, besonders bevorzugt in einem Lösemittel ausgewählt aus der Gruppe bestehend aus Dimethylformamid, Acetonitril, Dimethylacetamid und N-Methylpyrrolidinon, besonders bevorzugt Dimethylformamid aufgenommen. Pro Mol eingesetzte Verbindung der Formel (II) können zur Herstellung dieser Lösung beispielsweise 0,001 bis 10 L, bevorzugt 0,01 bis 5 L, besonders bevorzugt 0,05 bis 1 L Lösemittel verwendet werden. Vorzugsweise gelangen an dieser Stelle pro Mol ursprünglich eingesetzte Verbindung der Formel (II) etwa 0,07 bis 0,5 L Lösemittel zum Einsatz.

Die so erhaltene Lösung wird sodann über einen Zeitraum von beispielsweise 10 Minuten bis 3 Stunden, vorzugsweise 20 Minuten bis 2 Stunden zu gerührtem und auf eine Temperatur von mehr als 70°C, vorzugsweise mehr als 80°C, bevorzugt mehr als 90°C, höchstens aber 139°C erwärmtem Essigsäureanhydrid gegeben. Besonders bevorzugt wird das vorgelegte Essigsäureanhydrid auf eine Temperatur von etwa 120-135°C erwärmt. Pro Mol ursprünglich eingesetzte Verbindung der Formel (II) werden erfindungsgemäß beispielsweise 1 bis 10 Mol, bevorzugt 2 bis 8 Mol, besonders bevorzugt etwa 3 bis 6 Mol Essigsäureanhydrid verwendet. Erfindungsgemäß bevorzugt gelangen pro Mol eingesetzte Verbindung der Formel (II) etwa 4 bis 5 Mol Essigsäureanhydrid zur Anwendung. Während dieser Umsetzung wird CO₂ frei. Nach beendeter Zugabe der die Verbindung der Formel (IV) enthaltenden Lösung werden weitere beispielsweise 0,0005 bis 5 L, bevorzugt 0,005 bis 2,5 L, besonders bevorzugt 0,025 bis 0,5 L des vorstehend genannten Lösemittels zugegeben und die erhaltene Mischung bei konstanter Temperatur weitere 10 Minuten bis beispielsweise 6 Stunden, vorzugsweise weitere 30 Minuten bis 3 Stunden, besonders bevorzugt weitere 1 bis 2 Stunden gerührt. Anschließend werden alle flüssigen Bestandteile der Reaktionsmischung destillativ bei wenigstens 40°C, bevorzugt wenigstens 50°C, besonders bevorzugt bei etwa 55-70°C unter vermindertem Druck, vorzugsweise bei etwa 20 mbar oder weniger, bevorzugt bei etwa 10 mbar oder weniger entfernt.

Der verbleibende Rückstand wird sodann in einem geeigneten Lösemittel, vorzugsweise in Wasser und/oder einem niederen Alkohol, ausgewählt aus der Gruppe bestehend aus Methanol, Ethanol und Isopropanol, besonders bevorzugt Wasser, Ethanol oder ein Gemisch davon, aufgenommen. Pro Mol ursprünglich eingesetzte Verbindung der Formel (II) werden an dieser Stelle bevorzugt 0,1 bis 3 L, besonders bevorzugt etwa 0,5 bis 2 L eines der vorstehend genannten Alkohole im Gemisch mit beispielsweise 0,01 bis 1 L, bevorzugt 0,05 bis 0,5 L Wasser als Lösemittel verwendet.

Zur Verseifung der Esterfunktion der in nunmehr gelöster Form vorliegenden Verbindung der Formel (V) wird diese mit einer geeigneten Base versetzt. Als Base kommen bevorzugt anorganische Basen, ausgewählt aus der Gruppe der Alkali- oder Erdalkalicarbonate, Alkali- oder Erdalkalialkoholate und Alkali- oder Erdalkalihydroxyde in Betracht. Besonders bevorzugt sind dabei die Hydroxide des Lithiums, Natriums, Kaliums und Calciums besonders bevorzugt des Natriums oder Calciums. Erfindungsgemäß besonders bevorzugt wird Natriumhydroxid als Base verwendet. Vorstehend genannte Basen können in Reinform oder, besonders bevorzugt, in Form wässeriger konzentrierter Lösungen zum Einsatz gelangen. Wird beispielsweise die erfindungsgemäß besonders bevorzugte Base Natriumhydroxid verwendet, so erfolgt deren Zugabe vorzugsweise in Form wässeriger Lösungen mit einer Konzentration von wenigstens 40 Gew%. Pro Mol ursprünglich eingesetzter Verbindung der Formel (II) ist der Einsatz wenigstens stöchiometrischer Mengen an Base erforderlich. Allerdings ist auch die Verwendung der Base im Überschuss möglich. Vorzugsweise werden pro Mol ursprünglich eingesetzter Verbindung der Formel (II) etwa 1,1 bis 4 Mol, vorzugsweise 1,5 bis 3 Mol, besonders bevorzugt etwa 1,7 bis 2,5 Mol der vorstehend genannten Base eingesetzt. Die Zugabe der Base zur Lösung des Esters der Formel (V) kann beispielsweise bei einer Temperatur in einem Bereich von 0 bis 50°C erfolgen. Bevorzugt wird allerdings nach Zugabe der Base die erhaltene Reaktionsmischung auf eine Temperatur von oberhalb 50°C, besonders bevorzugt oberhalb 60°C erwärmt. In einer besonders bevorzugten Ausführungsform der vorliegenden Erfindung wird die erhaltene Reaktionsmischung nach vollständiger Zugabe der Base unter Rühren für einen Zeitraum von etwa 15 Minuten bis 4 Stunden, bevorzugt 30 Minuten bis 3 Stunden besonders bevorzugt 1 bis 2 Stunden bis zum Rückfluss erhitzt. Anschließend wird das Lösemittel destillativ bei wenigstens 40°C, bevorzugt wenigstens 50°C, besonders bevorzugt bei etwa 50-60°C unter vermindertem Druck, vorzugsweise bei etwa 80 mbar oder weniger, bevorzugt bei etwa 60 mbar oder weniger, besonders bevorzugt bei etwa 50 mbar oder weniger entfernt.

Der erhaltene Rückstand wird in Wasser aufgenommen. Hierbei gelangen pro Mol ursprünglich eingesetzte Verbindung der Formel (II) etwa 0,01 bis 1, bevorzugt etwa 0,1 bis 1 L Wasser zum Einsatz. Aus dieser Mischung wird das Tropenol mittels eines geeigneten, mit Wasser nicht mischbaren organischen Lösemittels, vorzugsweise mittels eines Lösemittels ausgewählt aus der Gruppe bestehend aus Toluol, Methyl-tert-butylether, Dichlormethan, Chloroform, vorzugsweise Dichlormethan extrahiert. Pro Mol eingesetzte Verbindung der Formel (II) werden erfindungsgemäß insgesamt etwa zwischen 0,5 und 5, bevorzugt zwischen 0,75 und 4 Litern organisches Lösemittel für die Extraktion verwendet. Die Extraktion wird erfindungsgemäß zwischen 3 und 8, bevorzugt 4 bis 6 mal durchgeführt. Nach beendeter Extraktion werden die organischen Phasen vereint und das organische Lösemittel im Vakuum abdestilliert.

Das verbleibende Rohprodukt wird in einem organischen Lösemittel, ausgewählt aus der Gruppe bestehend aus Methanol, Ethanol, Isopropanol, vorzugsweise Isopropanol aufgenommen. Pro Mol ursprünglich eingesetzte Verbindung der Formel (II) werden erfindungsgemäß zwischen 0,1 und 4,0 Litern, vorzugsweise zwischen etwa 1 und 2 Litern des vorstehend genannten Lösemittels verwendet. Die erhaltene Lösung wird gegebenenfalls filtriert. Das Filtrat enthält Tropenol der Formel (I) in Form seiner freien Base. Sofern die freie Base in die nächste Umsetzung eingesetzt werden soll, erfolgt an dieser Stelle das Abdestillieren des Lösemittels unter Vakuum. Die verbleibende freie Base ist dann, ohne weitere Reinigung, in die nächsten Syntheseschritte einsetzbar. Erfindungsgemäß bevorzugt wird die freie Base des Tropenols aber in eines der Säureadditionssalze überführt. Als Säureadditionssalze des Tropenols werden im Rahmen der vorliegenden Erfindung die Salze verstanden, die ausgewählt sind aus der Gruppe bestehend aus Hydrochlorid, Hydrobromid;, Hydrogenphosphat, Hydrogensulfat, Tetrafluoroborat oder Hexafluorophosphat. Besonders bevorzugt sind dabei die Salze Hydrobromid und Hydrochlorid, wobei dem Tropenolhydrochlorid erfindungsgemäß eine besondere Bedeutung zukommt. Zur Darstellung der Säureadditionssalze wird das Filtrat auf eine Temperatur im Bereich von -20°C bis 20°C, vorzugsweise im Bereich von -10°C und 15°C gekühlt. Die dadurch erhaltene Suspension wird anschließend mit der zur Bildung des Säureadditionssalzes Hydrochlorid, Hydrobromid', Hydrogenphosphat, Hydrogensulfat, Tetrafluoroborat oder Hexafluorophosphat erforderlichen entsprechenden Säure versetzt. Pro Mol ursprünglich eingesetzter Verbindung der Formel (II) sind wenigstens 1 Mol der jeweiligen Säure einzusetzen. Gegebenenfalls ist es im Rahmen der erfindungsgemäßen Verfahren möglich, die Säure im Überschuss (d.h. 1,1 bis etwa 2-3 Mol pro Mol ursprünglich eingesetzte Base (II)) zu verwenden. Erfindungsgemäß bevorzugt wird das Hydrochlorid des Tropenols hergestellt. Die Zugabe der dafür erforderlichen Salzsäure kann entweder in Form von Lösungen oder gasförmig erfolgen. Vorzugsweise wird Chlorwasserstoff in gasförmiger Form verwendet. Die Zugabe einer der vorstehend genannten Säuren zur Lösung der freien Base des Tropenols (I) erfolgt, bis ein pH von etwa 1 bis 5, vorzugsweise von etwa 1,5 bis 4 erreicht ist. Nach beendeter Zugabe der Säure kann bei konstanter Temperatur gegebenenfalls noch 0,5 bis 2 Stunden nachgerührt werden. Abschließend wird das ausgefallene Säureadditionssalz des Tropenols abgetrennt und gegebenenfalls mit einem Lösemittel, ausgewählt aus der Gruppe bestehend aus Aceton, Methylisobutylketon und Methylethylketon, vorzugsweise Aceton gewaschen und im Vakuum oder unter Inertgas (beispielsweise Stickstoff), gegebenenfalls bei erhöhter Temperatur getrocknet.

Wie einleitend formuliert, stellt Tropenol, welches gemäß dem erfindungsgemäßen Herstellverfahren zugänglich ist, eine wertvolle Ausgangsverbindung zur Darstellung therapeutisch wirksamer Verbindungen wie beispielsweise Tiotropiumbromid, Ipratropiumbromid oder BEA2108 dar. Aufgrund der hohen Reinheit, in der Tropenol gemäß der vorliegenden Erfindung zugänglich wird, ist es möglich die vorstehend genannten Wirkstoffe in den für die pharmazeutische Anwendung notwendigen Spezifikationen bereit zu stellen.

Dementsprechend zielt die vorliegende Erfindung ferner auf die Verwendung von Tropenol, gegebenenfalls in Form seiner Säureadditionssalze als Ausgangsmaterial zur Herstellung therapeutisch wirksamer Verbindungen wie beispielsweise Tiotropiumbromid, Ipratropiumbromid oder BEA2108, bevorzugt Tiotropiumbromid. Ferner betrifft die vorliegende Erfindung die Verwendung von Verbindungen der Formel (II) 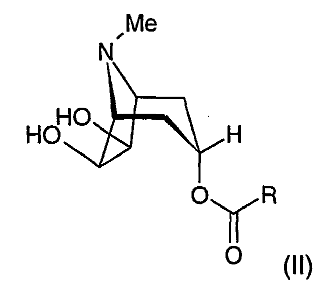 worin R die vorstehend genannten Bedeutungen haben kann, gegebenenfalls in Form ihrer Säureadditionssalze sowie gegebenenfalls in Form ihrer Hydrate, als Ausgangsmaterial zur Herstellung therapeutisch wirksamer Verbindungen wie beispielsweise Tiotropiumbromid, Ipratropiumbromid oder BEA2108, bevorzugt Tiotropiumbromid.

Bevorzugt betrifft die vorliegende Erfindung die Verwendung von Meteloidin, gegebenenfalls in Form seiner Säureadditionssalze, sowie gegebenenfalls in Form seiner Hydrate, als Ausgangsmaterial zur Herstellung therapeutisch wirksamer Verbindungen wie beispielsweise Tiotropiumbromid, Ipratropiumbromid oder BEA2108, bevorzugt Tiotropiumbromid.

Zur Darstellung von Tiotropiumbromid, ausgehend von Tropenol kann wie in Schema 1 dargestellt, vorgegangen werden.

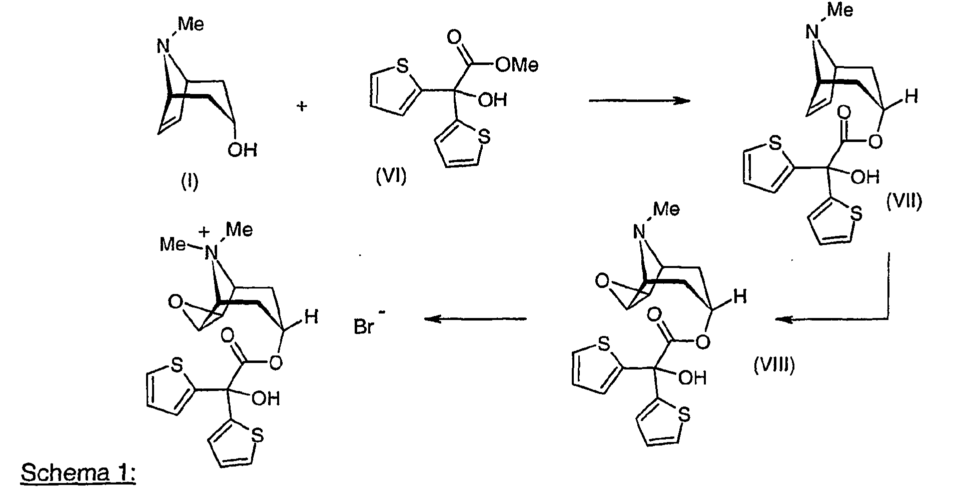

Ausgehend von gemäß dem erfindungsgemäß erhältlichen! Tropenol (I) erfolgt durch Umsetzung mit Di-(2-thienyl)-glykolsäurederivaten (Vl) zunächst die Bildung des Di-(2-thienyl)-glykolsäure-tropenolesters (VII). Dieser wird durch Epoxidierung der olefinischen Doppelbindung in den entsprechenden Scopinester (VIII) überführt, aus dem Tiotropiumbromid durch Umsetzung mit Methylbromid erhältlich wird.

Dementsprechend betrifft ein besonders bevorzugter Aspekt der vorliegenden Erfindung ein Verfahren zur Herstellung von Tiotropiumbromid 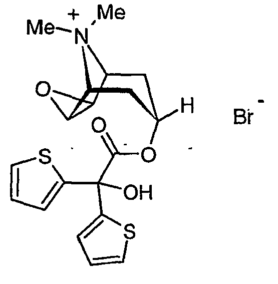 dadurch gekennzeichnet, dass in einem ersten Schritt eine Verbindung der Formel (II) 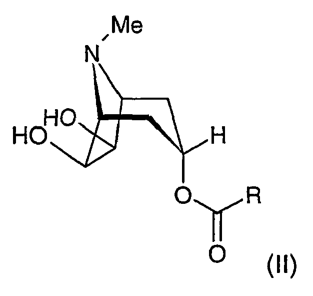 worin R die vorstehend genannten Bedeutungen haben kann gegebenenfalls in Form ihrer Säureadditionssalze sowie gegebenenfalls in Form ihrer Hydrate in einem geeigneten Lösemittel mit einem Formamid-acetal der Formel (111) 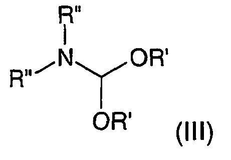 worin
R' und R" die vorstehend genannten Bedeutungen haben können, zu einer Verbindung der Formel IV 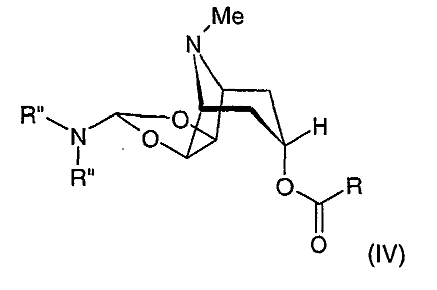 worin die Reste R, R' und R" die oben genannten Bedeutungen haben können, umsetzt, diese anschließend unter Decarboxylierung in einen Ester der Formel (V) 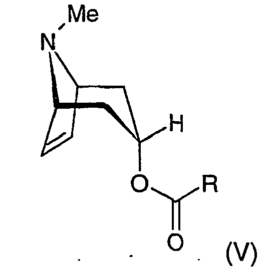 worin R die vorstehend genannten Bedeutungen haben kann, überführt und diesen zu Tropenol der Formel (I) verseift, dieses gegebenenfalls in Form seiner Säureadditionssalze, in einem zweiten Schritt mit einem Ester der Formel (VI) 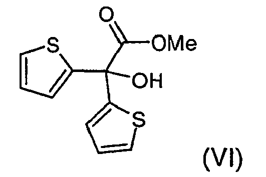 zum Tropenolester der Formel (VII) 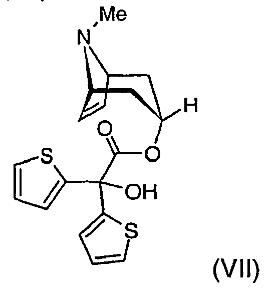 umgesetzt wird, dieser in einem dritten Schritt zum Scopinester der Formel (VIII) 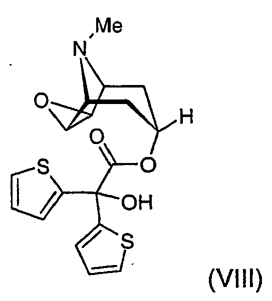 epoxidiert wird und dieser in einem vierten Schritt mittels Methylbromid zu Tiotropiumbromid quaternisiert wird.

Die nachfolgenden Beispiele dienen der Illustration exemplarisch durchgeführter Syntheseverfahren zur Herstellung von Tropenol sowie von daraus erhältlichem Tiotropiumbromid. Sie sind lediglich als mögliche, exemplarisch dargestellte Vorgehensweisen zu verstehen, ohne die Erfindung auf deren Inhalt zu beschränken.

### Beispiel 1:

### Darstellung von Tropenol (I) in Form seines Hydrochlorids

19,5 kg Dimethylformamiddimethylacetal werden in eine geeignet dimensionierte Rührwerkapparatur vorgelegt und portionsweise mit 20,9 kg Meteloidin versetzt. Nach beendeter Zugabe wird die erhaltene Mischung unter Rühren langsam auf eine Temperatur von etwa 80°C erwärmt. Das im Laufe der Reaktion frei werdende Methanol destilliert dabei ab. Nach beendeter Umsetzung wird das überschüssige Dimethylformamiddimethylacetal bei etwa 50-60°C unter vermindertem Druck (30mbar oder weniger) abdestilliert. Zum verbleibenden Rückstand werden anschließend 8 L Dimethylformamid gegeben und die erhaltene Lösung unter Rühren auf etwa 45-50°C abgekühlt. Diese Lösung wird sodann über einen Zeitraum von etwa 30-70 Minuten bei etwa 125-135°C zu 37,7 kg gerührtem, auf 125°C erwärmtem Essigsäureanhydrid gegeben. Dabei wird gasförmiges CO₂ frei. Nach beendeter Zugabe werden weitere 4 L Dimethylformamid zugegeben und die gesamte Reaktionsmischung weitere 1,5 Stunden bei etwa 125-135°C gerührt. Nach beendeter Reaktion werden alle flüssigen Bestandteile durch Erwärmen auf etwa 60°C unter vermindertem Druck (etwa 5 mbar oder weniger) abdestilliert. Der verbleibende Rückstand wird in 71 L Ethanol aufgenommen und unter Rühren auf etwa 25°C abgekühlt. Nach Zugabe von 8 L Wasser und weiteren 10 L Ethanol wird der erhaltenen Mischung 45%-ige Natronlauge (18,3 kg) zugesetzt. Unter Rühren wird die erhaltene Mischung für etwa 1,5 Stunden zum Rückfluss erhitzt. Das Lösemittel wird sodann bei etwa 50-60°C unter vermindertem Druck (etwa 40 mbar) abdestilliert und der verbleibende Rückstand in 31 L Wasser aufgenommen. Zur Extraktion des Produkts erfolgt die Zugabe von 62 L Methylenchlorid. Nach Abtrennen der organischen Phase wird die verbleibende wässerige Phase erneut 2 mal mit je 30 L Methylenchlorid und 3 mal mit je 21 L Methylenchlorid extrahiert. Die erhaltenen organischen Phasen werden vereint und das Lösemittel destillativ entfernt. Der verbleibende Rückstand wird anschließend in etwa 35 kg Isopropanol aufgenommen, mit 1,6 kg Clarcel versetzt, die erhaltene Mischung gerührt und filtriert. In die so erhaltene Lösung wird anschließend bei etwa -10°C bis +10°C Innentemperatur gasförmiger Chlorwasserstoff eingeleitet, bis ein pH-Wert von etwa 2-3 erreicht ist (ca. 3,0 kg HCl-Gas). Nach beendeter Gaseinleitung wird anschließend bei konstanter Temperatur etwa eine Stunde nachgerührt. Der entstandene Feststoff, Tropenol-hydrochlorid, wird abgetrennt und bei etwa 40-45°C unter Stickstoff getrocknet.
Ausbeute: 11,5 kg Tropenol-hydrochlorid (80% bezogen auf eingesetztes Meteloidin)

### Beispiel 2: Darstellung von Tiotropiumbromid

### a) Darstellunci des Tropenolesters (VII)

Zu 10,9 kg Tropenolhydrochlorid (erhältlich nach Beispiel 1) in Toluol (95 L) wird bei 25°C Ammoniak (1,8 kg) eingeleitet. Die erhaltene Suspension wird bei konstanter Temperatur etwa 1 h gerührt. Anschließend wird entstandenes Ammoniumchlorid abfiltriert und mit Toluol (26 L) nachgespült. Bei etwa 50°C Manteltemperatur wird ein Teil des Toluols (ca. 60 L) im Vakuum abdestilliert. Nach Abkühlen auf etwa 25°C werden 15,8 kg Di-(2-thienyl)-glykolsäuremethylester zugegeben und die erhaltene Mischung zum Lösen auf 50°C erwärmt. In einem weiteren Apparat wird Toluol (40 L) vorgelegt und in diese bei etwa 25°C Natriumhydrid (2,7 kg) eingetragen. In diese Lösung wird innerhalb von 1 h die zuvor generierte Lösung aus Tropenol und Di-(2-thienyl)-glykolsäuremethylester bei 30°C gegeben. Nach beendeter Zugabe wird bei reduziertem Druck etwa 7 Stunden unter Rühren auf 75°C erhitzt. Das sich bildende Methanol wird dabei abdestilliert. Die verbleibende Mischung wird abgekühlt und in eine Mischung von Wasser (958 L) und 36 %iger Salzsäure (13,2 kg) gegeben. Die wässrige Phase wird anschließend abgetrennt und mit Methylenchlorid (56 L) gewaschen. Nach erneuter Methylenchloridzugabe (198 L) wird das so erhaltene Gemisch mit vorbereiteter Sodalösung (9,6 kg Soda in 45 L Wasser) auf pH = 9 gestellt. Die Methylenchlorid-Phase wird abgetrennt und die wässerige Phase mit Methylenchlorid (262 L) ausgerührt. Die Methylenchlorid-Phase werden bei 65°C bis auf den Rückstand eingeengt. Der Rückstand wird in Toluol (166 L) aufgenommen und auf 95°C erhitz. Die toluolische Lösung wird auf 0°C abgekühlt. Die erhaltenen Kristalle werden abgetrennt, mit Toluol (33 L) gewaschen und bei etwa 50°C für max. 24 Std. im Stickstoffstrom getrocknet.
Ausbeute: 18,6 kg (83%);Schmp.: ca. 160°C (bestimmt über DSC bei Heizrate von 10K/min);

### b) Darstellung des Scopinesters (VIII)

In einem geeigneten Reaktionsapparat werden 260 L DMF vorgelegt und auf 50°C erwärmt. Anschließend werden 16,2 kg Tropenolester (IV) zugegeben und es wird gerührt, bis eine klare Lösung erhalten worden ist. Nach Abkühlen auf 40°C werden nacheinander und portionsweise Wasserstoffperoxid-Harnstoff-Komplex (10,2 kg), Wasser (13 L) und Vanadium-(V)-oxid (0,7 kg) eintragen und der Apparateinhalt auf etwa 50°C erhitzt. Nach 2 - 3 h Rühren bei konstanter Temperatur wird auf etwa 20°C abgekühlt. Das erhaltene Reaktionsgemisch wird mit Salzsäure (36 %ig) auf etwa pH 4,0 eingestellt. Vorbereitete Natriumbisulfit-Lösung (2,4 kg in 24 L Wasser) wird zugegeben. Bei einer Innentemperatur von 35°C wird das Lösemittel im Vakuum teilweise abdestilliert (etwa 210 L). Es wird wiederum auf etwa 20°C abgekühlt und mit Clarcel (3,2 kg) versetzt. Mit verdünnter Salzsäure (36%-ig, 0,8 kg in etwa 440 L Wasser) wird auf einen pH von etwa 2,0 eingestellt. Die erhaltene Lösung wird filtriert und mit Methylenchlorid (58 L) extrahiert. Die Methylenchloridphase wird verworfen. Zur wässrigen Phase wird erneut Methylenchlorid (130 L) gegeben und mit einer vorbereiteten Soda-Lösung (11,0 kg in 51 L Wasser) wird ein pH von etwa 10,0 eingestellt. Die Methylenchlorid-Phase wird abgetrennt und die wässrige Phase mit Methylenchlorid (136 L) extrahiert. Im schwachen Vakuum (600 - 700 mbar) wird bei 40°C von den vereinigten Methylenchloridphasen Methylenchlorid (etwa 175 L) abdestilliert. Der Apparateinhalt wird auf 20°C abgekühlt, Acetylchlorid (etwa 0,5 kg) wird zugesetzt und es wird für etwa 40 Minuten bei 20°C gerührt. Die Reaktionslösung wird in einen zweiten Apparat überführt. Mit einer vorbereiteten Salzsäure-Lösung (4,7 kg Salzsäure 36 %ig in 460 L Wasser) wird bei 20°C ein pH von 2,0 eingestellt. Die Methylenchlorid-Phase wird abgetrennt und entsorgt. Die wässrige Phase wird mit Methylenchlorid (39 L) gewaschen. Dann wird Methylenchlorid (130 L) zugesetzt und mit einer vorbereiteten Soda-Lösung (7,8 kg Soda in 38 L Wasser) bei 20°C ein pH von 10,0 eingestellt. Nach 15 Min. Rühren wird die organische Phase abgetrennt und die wässrige Phase zweimal mit Methylenchlorid (97 L und 65 L) gewaschen. Die Methylenchlorid-Phasen werden vereinigt und im schwachen Vakuum ein Teil des Methylenchlorids (90 L) bei einer Temperatur von 30 - 40°C abdestilliert. Anschließend wird Dimethylformamid (114 kg) zugesetzt und unter Vakuum bei 40°C das restliche Methylenchlorid abdestilliert. Der Apparateinhalt wird auf 20°C abgekühlt.

### c) Darstellung des Tiotropiumbromids

Zur nach vorhergehender Vorschrift erhältlichen Scopinester-Lösung wird bei 20°C Methylbromid (5,1 kg) eingeleitet. Der Anlageninhalt wird bei 30°C für etwa 2,5 Tage gerührt. Bei 50°C werden im Vakuum 70 L DMF abdestilliert. Die Lösung wird in einen kleineren Apparat überführt. Es wird mit DMF (10 L) nachgespült.. Bei 50°C wird im Vakuum weiteres DMF abdestilliert bis eine Gesamtdestillatmenge von etwa 100 L erreicht ist. Es wird auf 15°C abgekühlt und bei dieser Temperatur 2 h nachgerührt. Das Produkt wird mittels Nutschentrockner isoliert, mit 15°C kaltem DMF (10 L) und 15°C kaltem Aceton (25 L) gewaschen. Es wird bei max. 50°C für max. 36 Std. im Stickstoffstrom getrocknet. Ausbeute: 13,2 kg (88 %);
Schmp.: 200-230°C (abh. von Reinheitsgrad des Rohprodukts);

Das so erhaltene Rohprodukt (10,3 kg) wird in Methanol (66 L) eingetragen. Das Gemisch wird zum Lösen zum Rückfluss erhitzt. Die Lösung wird auf 7°C abgekühlt und 1,5 h bei dieser Temperatur nachgerührt. Das Produkt wird mittels Nutschentrockner isoliert, mit 7°C kaltem Methanol (11 L) gewaschen und max. 36 h bei etwa 50°C im Stickstoffstrom getrocknet. Ausbeute: 9,9 kg (96 %);
Schmp.: 228°C (bestimmt über DSC bei Heizrate von 10K/min).

Gegebenenfalls kann das so erhaltene Produkt in das kristalline Monohydrat des Tiotropiumbromids überführt werden. Hierzu kann wie folgt vorgegangen werden.
In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei etwa 25°C über etwa 2 Stunden im Stickstoffstrom getrocknet. Ausbeute : 13,4 kg Tiotropiumbromid-monohydrat (86 % d. Th.)
Schmp.: 230°C (bestimmt über DSC bei Heizrate von 10K/min).

## Patentansprüche

1. Verfahren zur Herstellung von Tropenol (I) 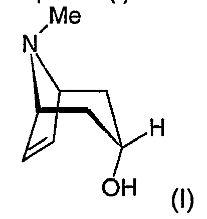 gegebenenfalls in Form seiner Säureadditionssalze, **dadurch gekennzeichnet, dass** eine Verbindung der Formel (II) 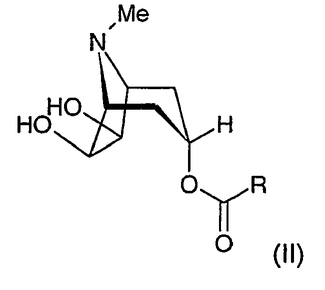 worin
R ein Rest ausgewählt aus C₁-C₄-Alkyl, C₂-C₆-Alkenyl. und C₁-C₄-alkylen-Phenyl, die jeweils substituiert sein können durch Hydroxy oder C₁-C₄₋Alkoxy, bedeutet,
gegebenenfalls in Form ihrer Säureadditionssalze sowie gegebenenfalls in Form ihrer Hydrate in einem geeigneten Lösemittel mit einem Formamid-acetal der Formel (III) 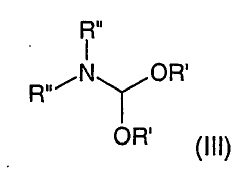 worin
R' C₁-C₄-Alkyl und R" ein Rest ausgewählt aus C₁-C₄-Alkyl und C₁-C₄₋alkylen-Phenyl bedeuten, zu einer Verbindung der Formel IV 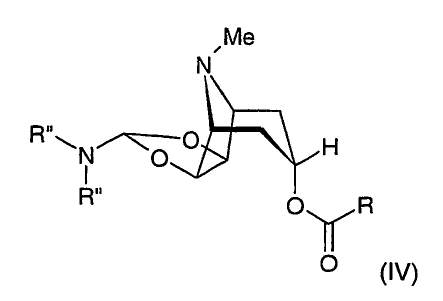 worin die Reste R, R' und R" die oben genannten Bedeutungen haben können, umgesetzt, diese anschließend unter Decarboxylierung in einen Ester der Formel (V) 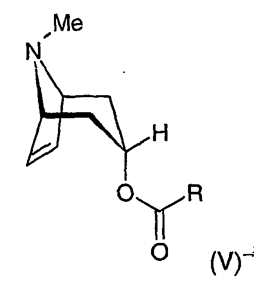 worin R die vorstehend genannten Bedeutungen-haben kann, überführt und diesen abschließend zur Verbindung der Formel (I) verseift und diese durch Umsetzung mit einer geeigneten Säure gegebenenfalls in ein Säureadditionssalz überführt wird.

2. Verfahren nach Anspruch 1, **dadurch gekennzeichnet, dass** eine Verbindung der Formel (II) worin R C₁-C₄-Alkyl oder C₂-C₄-Alkenyl bedeutet, gegebenenfalls in Form ihrer Säureadditionssalze sowie gegebenenfalls in Form ihrer Hydrate als Ausgangsmaterial verwendet wird und worin in dem eingesetzten Formamid-acetal der Formel (III) die Reste R' Methyl oder Ethyl und die Reste R" Methyl, Ethyl oder Propyl bedeuten.

3. Verfahren nach Anspruch 1 oder 2, **dadurch gekennzeichnet, dass** eine Verbindung der Formel (II) worin R 1-Propenyl, 2-Propenyl, 1-Buten-1-yl, 1-Buten-2-yl, 1-Buten-3-yl, 1-Buten-4-yl, 2-Buten-1-yl oder 2-Buten-2-yl, bevorzugt 2-Buten-2-yl bedeutet, gegebenenfalls in Form ihrer Säureadditionssalze sowie gegebenenfalls in Form ihrer Hydrate als Ausgangsmaterial verwendet wird und worin in dem eingesetzten Formamid-acetal der Formel (III) die Reste R' und R" Methyl oder Ethyl, bevorzugt Methyl bedeuten.

4. Verwendung von Verbindungen der Formel (II) 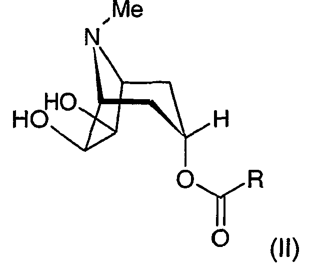 worin R die in den Ansprüchen 1 bis 3 genannten Bedeutungen haben kann, gegebenenfalls in Form ihrer Säureadditionssalze sowie gegebenenfalls in Form ihrer Hydrate, als Ausgangsmaterial zur Herstellung therapeutisch wirksamer Verbindungen.

5. Verwendung nach Anspruch 4, **dadurch gekennzeichnet, dass** es sich bei den therapeutisch wirksamen Verbindungen um Tiotropiumbromid, lpratropiumbromid oder BEA2108, bevorzugt Tiotropiumbromid handelt.

## Claims

1. Process for preparing tropenol (I) 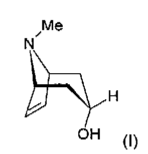 optionally in the form of the acid addition salts thereof, **characterised in that** a compound of formula (II) 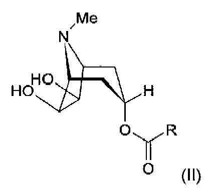 wherein
R denotes a group selected from C₁-C₄-alkyl, C₂-C₆-alkenyl and C₁-C₄-alkylene-phenyl, each of which may be substituted by hydroxy or C₁-C₄-alkoxy,
optionally in the form of the acid addition salts thereof as well as optionally in the form of the hydrates thereof, is reacted in a suitable solvent with a formamide-acetal of formula (III) 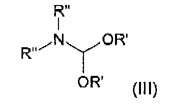 wherein
R' denotes C₁-C₄-alkyl and R" denotes a group selected from C₁-C₄₋alkyl and C₁-C₄-alkylene-phenyl, to obtain a compound of formula IV 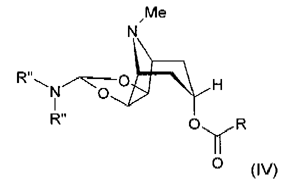 wherein the groups R, R' and R" may have the meanings given above, this is then converted by decarboxylation into an ester of formula (V) 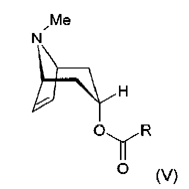 wherein R may have the meanings given above, and lastly this ester is saponified to obtain the compound of formula (I) which is optionally converted into an acid addition salt by reaction with a suitable acid.

2. Process according to claim 1, **characterised in that** a compound of formula (II) wherein R denotes C₁-C₄-alkyl or C₂-C₄-alkenyl, is used as starting material, optionally in the form of the acid addition salts thereof as well as optionally in the form of the hydrates thereof, and wherein in the formamide-acetal of formula (III) used the groups R' represent methyl or ethyl and the groups R" represent methyl, ethyl or propyl.

3. Process according to claim 1 or 2, **characterised in that** a compound of formula (II) wherein R denotes 1-propenyl, 2-propenyl, 1-buten-1-yl, 1-buten-2-yl, 1-buten-3-yl, 1-buten-4-yl, 2-buten-1-yl or 2-buten-2-yl, preferably 2-buten-2-yl, is used as starting material, optionally in the form of the acid addition salts thereof as well as optionally in the form of the hydrates thereof, and wherein in the formamide-acetal of formula (III) used the groups R' and R" represent methyl or ethyl, preferably methyl.

4. Use of compounds of formula (II) 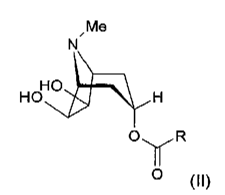 wherein R may have the meanings given in claims 1 to 3, optionally in the form of the acid addition salts thereof as well as optionally in the form of the hydrates thereof, as starting material for preparing therapeutically active compounds.

5. Use according to claim 4, **characterised in that** the therapeutically active compounds are tiotropium bromide, ipratropium bromide or BEA2108, preferably tiotropium bromide.

## Revendications

1. Procédé de préparation du tropénol (I) 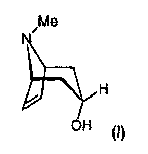 éventuellement sous forme de ses sels d'addition d'acide, **caractérisé en ce qu'**un composé de formule (II) 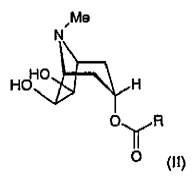 où
R représente un groupement choisi parmi C₁-C₄-alkyle, C₂-C₆-alcényle et C₁-C₄₋alkylènephényle, qui peuvent être substitués dans chaque cas par hydroxy ou C₁₋C₄-alcoxy,
éventuellement sous forme de ses sels d'addition d'acide ainsi que éventuellement sous forme de ses hydrates est converti dans un solvant approprié avec un formamide-acélal de formée (III) 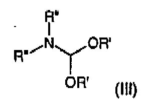 où
R' représente C₁-C₄-alkyle et R" représente un groupement choisi parmi C₁-C₄₋alkyle et C₁-C₄-alkylènephényle, en un composé de formule IV 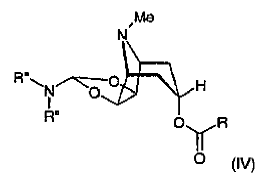 où les groupements R, R' et R" peuvent avoir les significations citées ci-dessus, celui-ci est ensuite converti par décarboxylation en un ester de formule (V) 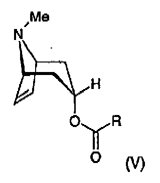 où R peut avoir les significations citées précédemment, et celui-ci est finalement saponifié en le composé de formule (I) et celui-ci est éventuellement converti en un sel d'addition d'acide par réaction avec un acide approprié.

2. Procédé selon la revendication 1 **caractérisé en ce qu'**un composé de formule (II) où R représente C₁-C₄-alkyle ou C₂-C₄-alcényle, éventuellement sous forme de ses sels d'addition d'acide ainsi que éventuellement sous forme de ses hydrates est utilisé comme produit de départ et où, dans le formamide-acétal de formule (III) utilisé, les groupements R' représentent méthyle ou éthyle et les groupements R" représentent méthyle, éthyle ou propyle.

3. Procédé selon la revendication 1 ou 2 **caractérisé en ce qu'**un composé de formule (II) où R représente 1-propényle, 2-propényle, 1-butén-1-yle, 1-butén-2-yle, 1-butén-3-yle, 1-butén-4-yle, 2-butén-1-yle ou 2-butén-2-yle, de préférence 2-butén-2-yle, éventuellement sous forme de ses sels d'addition d'acide ainsi que éventuellement sous forme de ses hydrates est utilisé comme produit de départ et où, dans le formamide-acétal de formule (III) utilisé, les groupements R' et R" représentent méthyle ou éthyle, de préférence méthyle.

4. Utilisation de composés de formule (II) 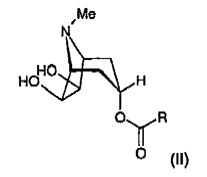 où R peut avoir les significations citées dans les revendications 1 à 3, éventuellement sous forme de leurs sels d'addition d'acide ainsi que éventuellement sous forme de leurs hydrates, comme produit de départ pour la préparation de composés thérapeutiquement eflicaces.

5. Utilisation selon la revendication 4 **caractérisée en ce qu'**il s'agit, concernant les composés thérapeutiquement efficaces, du bromure de thiotropium, du bromure d'ipratropium ou de BEA2108, de préférence du bromure de tiotropium.
